# EUROPEAN PATENT SPECIFICATION

(11) **EP 4 051 329 B1**
(45) Date of publication and mention of the grant of the patent: **04.03.2026**
(21) Application number: 20811178.1
(22) Date of filing: 27.10.2020
(51) Int. Cl.: A61L 29/08, A61L 29/14

(54) **URINARY CATHETERS AND METHODS FOR PREVENTING BACTERIAL INFECTIONS**
HARNKATHETER UND VERFAHREN ZUM VORBEUGEN BAKTERIELLER INFEKTIONEN
CATHÉTERS URINAIRES ET PROCÉDÉS POUR PRÉVENIR DES INFECTIONS BACTÉRIENNES

(30) Priority: 28.10.2019 US 201962926900 P
(43) Date of publication of application: 07.09.2022
(73) Proprietor: Hollister Incorporated, Libertyville, IL 60048-3781 (US)
(72) Inventor: SILEIKA, Tadas, S., Libertyville, IL 60048 (US); DEAN, Nicole, L., Libertyville, IL 60048 (US); PETTIGREW, Jacqueline, MJ, Libertyville, IL 60048 (US)
(74) Representative: FRKelly
(86) International application number: PCT/US2020/057514
(87) International publication number: WO 2021/086843

(56) References cited:
- EP-A1- 2 338 535
- EP-A1- 2 468 319
- WO-A1-2013/180585
- WO-A2-2014/203075
- US-A1- 2010 198 195
- US-A1- 2012 203 211

## Description

### TECHNICAL FIELD

The present disclosure generally relates to urinary catheters and methods for preventing bacterial infections, and more particularly, to catheters and methods for preventing urinary tract infections. The present disclosure also relates to urinary catheters and methods that inhibit the motility and swarming of bacteria within the urethra.

### BACKGROUND

It is desirable for urinary catheters to have a lubricated or lubricious outer surface to increase comfort of the patient during insertion into and/or removal from the body. One method for rendering the surface of a urinary catheter lubricious is to coat at least the insertable portion of the catheter with a lubricious hydrophilic coating. Another method is to apply a lubricant to the urinary catheter. Lubricity of the catheter minimizes soft tissue damage and reduces overall discomfort during use of the medical device. US 2010/0198195 discloses a urinary catheter with a polymeric coating, wherein the coating includes hydrophilic polymers, hydrophobic polymers, and mixtures of these two types of polymers.

Although catheters are typically prepared in sterile environments and are provided with safe handling instructions, catheter users are at risk of contracting a urinary tract infection due to several different factors. For example, the catheter may become contaminated during use and introduce bacteria into the urethra. The catheter also may carrier bacteria along the urinary tract. Additionally, because urine is voided from the bladder through the catheter, urine does not flow directly through the urethra. Thus, urine is no longer a factor in flushing out the urethra or wetting of the urethral tissue.

Therefore, there is a need for catheters, which reduce the risk of urinary tract infections.

### SUMMARY

There are several aspects of the present subject matter, which may be embodied separately or together in the devices and systems described and claimed below. These aspects may be employed alone or in combination with other aspects of the subject matter described herein, and the description of these aspects together is not intended to preclude the use of these aspects separately or the claiming of such aspects separately or in different combinations as set forth in the claims appended hereto.

In one aspect, a urinary catheter product for insertion into the urethra, according to claim 1.
The catheter product includes a catheter, and a compound that impedes the mobility and/or swarming of bacteria.

### BRIEF DESCRIPTION OF THE DRAWINGS

Fig. 1 is a plan view of one embodiment of the present disclosure; and
Fig. 2 is a plan view of one embodiment of a catheter of the present disclosure.

### DETAILED DESCRIPTION OF THE ILLUSTRATED EMBODIMENTS

The embodiments disclosed herein are for the purpose of providing a description of the present subject matter, and it is understood that the subject matter may be embodied in various other forms and combinations not shown in detail. Therefore, specific embodiments and features disclosed herein are not to be interpreted as limiting the subject matter as defined in the accompanying claims.

For catheter users, whether in-dwelling or intermittent, urine is no longer a factor that participates in regular flushing or wetting of the urethra. Because the users are unable to void directly through the urethra, the urethra no longer experiences the continuous flushing and re-wetting of the tissues that normally occurs during active voluntary urination. When the mechanism fails, the risk of urinary tract infections increases.

Urinary tract infections can be a result of bacteria swarming and/or traveling up through the urethra towards the bladder. Traveling of bacteria up the urethra is typically not a concern for those who can perform voluntary urination, because normal voiding tends to wash out and eliminate the bacteria from urethra, thereby prohibiting it from traveling toward the bladder and causing infection.

There are certain species of bacteria that are considered good "swimmers," which swarm and move when they receive certain signals from the surrounding environment. These bacteria are capable of movement even on solid surfaces through excretion of their own extracellular fluids. These motile bacteria also are known to become more pathogenic and increase their motility organelles, such as flagella (a process called hyperflagellation), when sensing certain signals, such as decreased viscosity in the fluids and mucus in their surrounding environment and/or an increase in chemotactants.

In a non-voiding patient, the urethral environment will be fairly viscous, due to the presence of mucosal secretions. When a hydrophilic catheter or a catheter lubricated with a water-based gel is introduced into the urethra, such introduction will likely deposit additional moisture, diluting the secretions, thus decreasing the overall viscosity of the urethral mucus and the surrounding urethral environment. This decrease in the urethral mucus viscosity provides a signal that activates the motility of the bacteria.

Furthermore, when a catheter is inserted into the urethra, the catheter, hydrophilic coating, hydration medium and/or lubricants may include a chemotactant that promotes chemotaxis (the movement of the bacteria in response to a chemical stimulant). For example, the hydrophilic coating and hydration medium may include a chemotactant, such as a polyol or polysaccharide, which stimulates the bacteria to move toward the chemotactant. If the catheter distributes this chemotactant at or near the bladder, the bacteria may be stimulated to undesirably swarm and move toward the bladder.

The catheter products disclosed herein impede bacteria mobility and/or mitigate the risk of bacteria swarming and traveling proximally up the urethra toward the bladder. The catheter products include a compound that physically and/or chemically impedes bacterial movement and/or provides a physical and/or chemical barrier to bacterial movement. Thus, when bacteria are stimulated to swarm and move by signals from changes in the urethral environment, the compounds will physically and/or chemically impede the movement and/or swarming of the bacteria. The compounds that impede the movement and/or swarming of the bacteria are delivered into the urethra by the catheter.

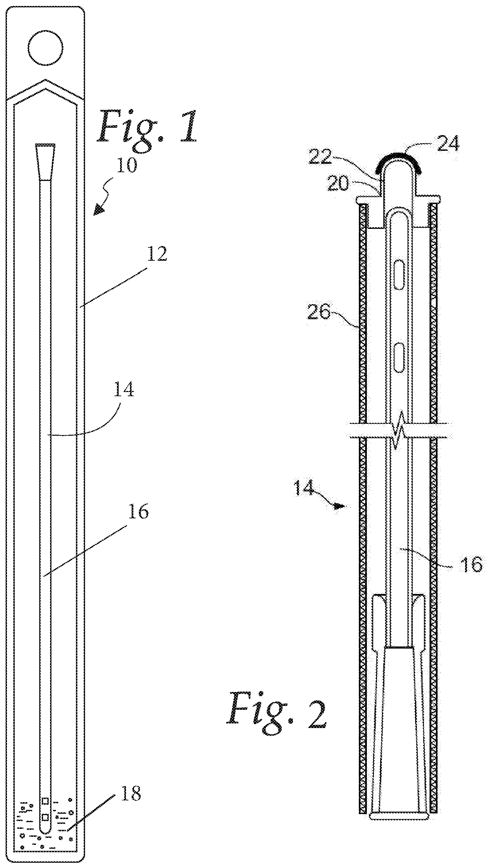

The compound that physically and/or chemically impedes the movement and/or swarming of the bacteria may be included in any component of a catheter product. For example, referring to Fig. 1, a catheter product 10 includes a package 12 including a catheter 14 having a catheter shaft 16. The outer surface of the catheter shaft 16 is hydrophilic. For example, the catheter 16 may be coated with a hydrophilic coating. The hydrophilic surface of the catheter shaft 16 becomes lubricious when wetted with a hydration medium 18 (water saline, or another polar solvent). In one alternative, the compound that physically and/or chemically impedes the movement and/or swarming of the bacteria may be contained in the hydrophilic material (e.g., the hydrophilic coating) and/or the hydration medium. When the catheter product includes a lubricating gel, the compound may be included in the lubricating gel. When the catheter is inserted into and through the urethra, the compound may be distributed along the urethra, where it will come into contact with bacteria.

Referring to Fig. 2, when the catheter product includes an introducer aid 20 that may be inserted into the urethral opening and which the catheter is inserted through, the introducer aid 20 or the tip 22 thereof may include the compound 24 that physically and/or chemically impedes the movement and/or swarming of the bacteria. When the catheter shaft 16 is inserted through the introducer aid 20, the retaining and/or viscosity increasing compound may be disposed onto the catheter shaft 16 wherein the catheter distributes the compound along the urethra. Additionally, a sleeve 26 may be attached to the introducer aid, wherein the sleeve surrounds the catheter.

In one embodiment, the mobility and/or swarming impeding compound can be incorporated into the catheter, hydration medium or in the hydrophilic coating of the catheter. The compound also may be incorporated into a lubricant that lubricates the catheter.

Such compounds include positively charged polysaccharides that may physically and electrostatically entrap bacteria, thereby preventing movement and/or swarming. In one example, the compound may be a polysaccharide, such as chitosan, that has a molecular weight between about 10k MW and 100k MW. Optionally, the polysaccharide also may have an 80% or greater degree of deacetylation. Additionally, the compounds are deposited in the urethra and may remain therein over a period of time. Furthermore, in intermittent catheterization, wherein catheterization is preformed several times a day, the compound may be redeposited or replenished several times a day with each catheterization procedure. Because the compounds may be in the urethra over a long period of time, the compounds can offer long-term antimicrobial properties, actively killing pathogenic bacteria over longer time scales.

In one embodiment, the compound may be chitosan, wherein the chitosan is incorporated into a hydration medium (such as water) for hydrating a hydrophilic coating of the catheter. Chitosan also may be incorporated into a lubricant for lubricating a catheter. The chitosan may form a hydrogel that creates a barrier to bacteria motility and swarming. The hydrogel is located on the surface of the catheter and is deposited along the urethra as the catheter is inserted into and withdrawn from the urethra. When deposited in the urethra, the hydrogel may physically entrap bacteria and/or entrap the bacteria by positive and negative charge interactions. For example, chitosan is positively charged at physiological and acidic pH, whereas certain bacteria outer surfaces are negatively charged. Thus, the bacteria is attracted to the chitosan and electrostatically entrapped by the chitosan. Furthermore, residual chitosan remaining in the urethra also may function to actively killing bacteria over longer time scales.

When it is desired to entrap bacteria with a positively charged surface, a negatively charged polysaccharide may be incorporated into the catheter product to provide a barrier to mobility that physically and/or electrostatically entraps positively charged bacteria.

When it is desired to inhibit motility of bacteria with a negatively charged surface, a negatively charged polysaccharide may be incorporated into the catheter product to impede motility through electrostatic repulsion.

In another embodiment, the hydration medium or a lubricant may include hyaluronic acid. The hyaluronic acid could be at a concentration of about 1 wt% of the hydration medium or lubricant. As hyaluronic acid is deposited within the urethra, it remains as a physical hydrogel and physically entraps, thereby preventing the bacteria from swarming and/or moving.

In one embodiment, the residual active compounds left behind in the urethra, such as the polysaccharides, will have longer time scales available to enact direct antimicrobial properties, such as killing of the microbes.

It will be understood that the embodiments described above are illustrative of some of the applications of the principles of the present subject matter.

## Claims

1. A urinary catheter product for insertion into a urethra, the catheter product comprising:
a catheter comprising a catheter shaft having a hydrophilic outer surface that becomes lubricious when hydrated;
a hydration medium for hydrating the hydrophilic outer surface;
a positively charged polysaccharide contained with the hydration medium; and
wherein the positively charged polysaccharide forms a hydrogel located on the hydrophilic outer surface of the catheter and the catheter deposits the hydrogel along the urethra as the catheter is inserted into and withdrawn from the urethra, and the hydrogel impedes motility and/or swarming of bacteria by electrostatically entrapping the bacteria.

2. The urinary catheter product of claim 1, wherein the hydrophilic outer surface of the catheter shaft comprises a hydrophilic coating.

3. The urinary catheter product of claim 1, wherein the polysaccharide is also contained in the hydrophilic outer surface.

4. The urinary catheter of any one of claims 1-3, wherein the polysaccharide comprises a molecular weight between about 10k MW and about 100k MW.

5. The urinary catheter product of any one of claims 1-4, wherein the polysaccharide has an 80% or greater degree of deacetylation.

6. The urinary catheter product of any one of the preceding claims, wherein the polysaccharide comprises chitosan.

7. The urinary catheter product of any one of the preceding claims, wherein the hydrogel also forms a physical barrier to bacteria movement and/or swarming.

8. The urinary catheter product of any one of the preceding claims, wherein the hydrogel also physically entraps bacteria.

## Patentansprüche

1. Harnkatheterprodukt zur Einführung in eine Harnröhre, wobei das Katheterprodukt Folgendes umfasst:
einen Katheter, umfassend einen Katheterschaft mit einer hydrophilen Außenfläche, die beim Hydratisieren gleitend wird;
ein Hydratationsmedium zum Hydratisieren der hydrophilen Außenfläche;
ein mit dem Hydratationsmedium enthaltenes positiv geladenes Polysaccharid; und
wobei das positiv geladene Polysaccharid ein sich auf der hydrophilen Außenfläche des Katheters befindendes Hydrogel bildet und der Katheter das Hydrogel beim Einführen des Katheters in die und Herausziehen aus der Harnröhre entlang der Harnröhre abscheidet und das Hydrogel Motilität und/oder Schwärmen von Bakterien durch elektrostatisches Einfangen der Bakterien behindert.

2. Harnkatheterprodukt nach Anspruch 1, wobei die hydrophile Außenfläche des Katheterschafts eine hydrophile Beschichtung umfasst.

3. Harnkatheterprodukt nach Anspruch 1, wobei das Polysaccharid auch in der hydrophilen Außenfläche enthalten ist.

4. Harnkatheter nach einem der Ansprüche 1-3, wobei das Polysaccharid ein Molekulargewicht zwischen etwa 10 k MW und etwa 100 k MW umfasst.

5. Harnkatheterprodukt nach einem der Ansprüche 1-4, wobei das Polysaccharid einen Deacetylierungsgrad von 80 % oder mehr aufweist.

6. Harnkatheterprodukt nach einem der vorhergehenden Ansprüche, wobei das Polysaccharid Chitosan umfasst.

7. Harnkatheterprodukt nach einem der vorhergehenden Ansprüche, wobei das Hydrogel auch eine physische Barriere gegenüber Bewegung und/oder Schwärmen von Bakterien bildet.

8. Harnkatheterprodukt nach einem der vorhergehenden Ansprüche, wobei das Hydrogel Bakterien auch physisch einfängt.

## Revendications

1. Dispositif de cathéter urinaire destiné à être inséré dans l'urètre, le dispositif comprenant :
un cathéter comprenant une tige de cathéter présentant une surface externe hydrophile qui devient lubrifiante lorsqu'elle est hydratée ;
un milieu hydratant destiné à hydrater la surface externe hydrophile ;
un polysaccharide chargé positivement contenu dans le milieu hydratant ; et
dans lequel le polysaccharide chargé positivement forme un hydrogel situé sur la surface externe hydrophile du cathéter et le cathéter dépose l'hydrogel le long de l'urètre lorsque le cathéter est inséré et retiré de l'urètre, et l'hydrogel empêche la motilité et/ou l'essaimage des bactéries en piégeant électrostatiquement les bactéries.

2. Produit de cathéter urinaire selon la revendication 1, dans lequel la surface externe hydrophile de la tige de cathéter comprend un revêtement hydrophile.

3. Produit de cathéter urinaire selon la revendication 1, dans lequel le polysaccharide est également contenu dans la surface externe hydrophile.

4. Cathéter urinaire selon l'une quelconque des revendications 1 à 3, dans lequel le polysaccharide comprend un poids moléculaire compris entre environ 10 kMW et environ 100 kMW.

5. Produit de cathéter urinaire selon l'une quelconque des revendications 1 à 4, dans lequel le polysaccharide présente un degré de désacétylation de 80 % ou plus.

6. Produit de cathéter urinaire selon l'une quelconque des revendications précédentes, dans lequel le polysaccharide comprend du chitosane.

7. Produit de cathéter urinaire selon l'une quelconque des revendications précédentes, dans lequel l'hydrogel forme également une barrière physique contre le mouvement et/ou l'essaimage des bactéries.

8. Produit de cathéter urinaire selon l'une quelconque des revendications précédentes, dans lequel l'hydrogel piège également physiquement des bactéries.
